Europäisches Patentamt

European Patent Office

Office européen des brevets

(11) Veröffentlichungsnummer: **0 209 030**
**A2**

## (12) EUROPÄISCHE PATENTANMELDUNG

(21) Anmeldenummer: 86109208.8

(51) Int. Cl.⁴: **A61K 37/02**

(22) Anmeldetag: 05.07.86

---

(30) Priorität: 19.07.85 DE 3525750

(43) Veröffentlichungstag der Anmeldung:
21.01.87 Patentblatt 87/04

(84) Benannte Vertragsstaaten:
**AT BE CH DE FR GB IT LI NL SE**

(71) Anmelder: **BASF Aktiengesellschaft**
**Carl-Bosch-Strasse 38**
**D-6700 Ludwigshafen(DE)**

(72) Erfinder: **Kirchner, Holger, Prof. Dr.**
**Kussmaulstrasse 3**
**D-6900 Heidelberg(DE)**
Erfinder: **Jacobsen, Helmut, Dr.**
**Kurpfalzstrasse 9**
**D-6905 Schriesheim(DE)**

---

(54) **Verwendung vom Tumornekrosefaktor zur Herstellung von Arzneimitteln.**

(57) Es wird die Verwendung von TNF bei der Bekämpfung von durch Virusinfektion verursachten Krankheiten beschrieben.

EP 0 209 030 A2

## Verwendung von TNF zur Herstellung von Arzneimitteln

TNF (Tumornekrosefaktor) ist eine Tumor zerstörende Substanz, die zur Bekämpfung maligner Tumoren Verwendung finden soll. Die Struktur dieser Substanz ist in Nature 312, 724 (1984) beschrieben worden.

Es wurde nun gefunden, daß sich TNF auch zur Behandlung viraler Infektionskrankheiten eignet.

Gegenstand der Erfindung ist die Verwendung von TNF zur Herstellung von Arzneimitteln mit antiviraler Wirksamkeit bzw. die Verwendung von TNF bei der Bekämpfung von durch Virusinfektion verursachten Krankheiten.

Als durch Virusinfektion verursachte Krankheiten sind insbesondere zu nennen: Schnupfen (Coryza Acuta), Coxsackie-Erkrankungen (wie Sommergrippe), Gelbfieber, Hepatitis, Herpes Simplex, Herpes zoster, Influenza, Keratoconjunctivitis epidemica, Lymphadenitis virale, Lymphopathia venera, Mononucleosis infectiosa, Morbilli (Masern), Parotitis epidemica, atypische Pneumonie, Poliomyelitis epidemica, Rabies, Rubella (Röteln), Trachom, Varicella (Windpocken), Variola (Pocken).

Da der TNF ein Polypeptid darstellt, welches im Magen-Darm-Trakt zerstört wird, kann er nur parenteral, vorzugsweise intravenös, verabfolgt werden. Hierzu eignen sich sterile isotonische Lösungen. Diese lassen sich beispeilsweise herstellen, indem man den TNF in einer blutisotonischen wäßrigen Lösung löst, die Lösung steril filtriert und in Ampullen abfüllt. Der pH-Wert der Lösung liegt vorzugsweise zwischen 5 und 8, insbesondere bei etwa 7,5.

Die zu applizierende Dosis liegt bei 0,001-0,02, vorzugsweise 0,002-0,008 mg TNF pro kg Körpergewicht und Tag. Die Behandlungsdauer beträgt in der Regel 2-6 Tage.

Die antivirale Wirksamkeit des TNF ließ sich beispielsweise wie folgt zeigen:

HEP-2-Zellen wurden in drei 96-Loch-Platten gezüchtet, bis sich ein konfluenter Zellrasen gebildet hatte. Die Zellen einer Platte wurden 18 h im üblichen Gewebkulturmedium (RPMI 1640) bei 37°C im Brutschrank mit 1 ng/ml TNF inkubiert. Nach 18 h wurden die Zellen mit Medium gewaschen und die TNF-Kultur und eine weitere Kultur mit Vesikular Stomatitis Virus (Virus Stamm Indiana) infiziert, und zwar mit je einem infektiösen Viruspartikel pro etwa 10 Zellen. Die Platten wurden wiederum für 48 h im Brutschrank inkubiert und die Zellen mit Kristallviolett angefärbt. Dabei wiesen die Zellen ohne TNF-Behandlung einen 100%igen zytopathischen Effekt auf, d.h. alle Zellen waren durch das Virus zerstört und farblos, während die Zellen, die mit TNF vorbehandelt waren, intakt und gefärbt waren.

Herstellung einer Applikationsform

100 mg TNF werden in 300 ml 20 mM Natriumphosphatpuffer pH 7,5 gelöst. Die Lösung wird mit Kochsalz blutisotonisch eingestellt, über einen Porenfilter (Porengröße 0,1-0,2 μ) steril filtriert und jeweils 1 ml steril in Ampullen gefüllt.

## Ansprüche

Verwendung von TNF zur Herstellung von Arzneimitteln zur Bekämpfung von durch Virusinfektion verursachten Krankheiten.